# EUROPEAN PATENT APPLICATION

(11) **EP 2 397 143 A1**
(43) Date of publication of application: **21.12.2011**
(21) Application number: 09814066.8
(22) Date of filing: 17.09.2009
(51) Int. Cl.: A61K 31/529, A61K 9/19, A61K 47/02, A61K 47/36, A61P 25/36, A61P 25/04

(54) **FREEZE-DRIED PREPARATION OF TETRODOTOXIN AND THE PRODUCING METHOD THEREOF**

(30) Priority: 17.09.2008 CN 200810211203
(71) Applicant: Xiamen Zhaoyang Biological Engineering Co., Ltd., Xiamen, Fujian 361005 (CN); The Third Institute Of Oceanography, State Oceanic, Xiamen, Fujian 361005 (CN)
(72) Inventor: YI, Ruizao, Xiamen Fujian 361005 (CN); CHEN, Hui, Xiamen Fujian 361005 (CN); HONG, Bihong, Xiamen Fujian 361005 (CN); XIE, Rongwei, Xiamen Fujian 361005 (CN); CHEN, Weizhu, Xiamen Fujian 361005 (CN); HONG, Zhuan, Xiamen Fujian 361005 (CN); XU, Shuzhen, Xiamen Fujian 361005 (CN)
(74) Representative: Laudens
(86) International application number: PCT/CN2009/074006
(87) International publication number: WO 2010/031346

(57) **Abstract**

A stable freeze-dried powder preparation of tetrodotoxin and the producing method thereof. The freeze-dried powder preparation has tetrodotoxin as the main active ingredient, and comprises solubilizer, excipient and stabilizer. The said solubilizer is citricacid. The excipient is sodium chloride, mannitol or their composite. The stabilizer is dextran, trehalose or their composite. The ratio of tetrodotoxin, excipient and stabilizer is 1:150-3000:50-500 or 50-6000. Preferably, the preparation comprises lidocaine hydrochloride as function modulator. The preparation of the present invention can be used for avoiding the dependent abstinence syndrome of drugs such as opiates and cannabis.

## Description

### BACKGROUND OF THE INVENTION

### 1. FIELD OF THE INVENTION

The invention relates to freeze-dried powder preparation of the TTX and the producing method thereof. More specifically, the invention relates to the freeze-dried preparation of stabilizer and excipient with appropriate formation framework using accurate fixed quantity of TTX as the main active ingredient. The freeze-dried powder preparation of the TTX and the producing method thereof can be used for avoiding the dependent abstinence syndrome of opiates such as ices and heroin, and amphetamine-type drugs with rapid onset effect and remarkable effect. It has swift and significant effect in cutting off the dependence for drugs. Moreover, the freeze-dried preparation of the TTX is stable and safe and has little irritation to the human body.

### 2. DESCRIPTION OF THE RELATED ART

Tetrodotoxin is a natural nonprotein neurotoxin. Its chemical structure is as follows: 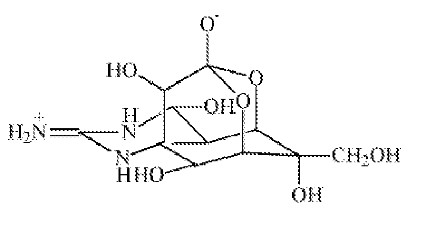 Chemical name: Octahydro-12-(hydroxymethyl)-2-imino-5,9: 7,10a-dimethano-10aH-[1,3] dioxocino[6,5-d]pyrimidine-4,7,10,11,12-pentol. Its English name is tetrodotoxin and is abbreviated as TTX.

Research shows that TTX can be used for avoiding the dependent abstinence syndrome of opiates such as ices and heroin and amphetamine-type drugs. It can also be used to control drug addiction seizures and eliminate abstinence response, and at the same time not produce dependence after stopping taking of drugs.

The TTX crystal is comparatively stable and can basically maintain quality for six months under the condition of 40°C (see Table 1). However, in water solution tetrodotoxin is highly sensitive to temperature and can easily degrade, faster with higher temperature. At 40°C, the regular tetrodotoxin injection solution would degrade from 99.20% to 65.57% in content after standing for 30 days, a significant degrade of 33.63% (see Table 2).

**Table 1: Stability test result of tetrodotoxin under high temperature of 40°C***

| Time | 0 month | 1 month | 2 months | 3 months | 6 months |
|---|---|---|---|---|---|
| Content (%) | 100.45 | 99.16 | 99.90 | 100.68 | 99.52 |
| Related material | <Reference solution peak content | < Reference solution peak content | < Reference solution peak content | < Reference solution peak content | < Reference solution peak content |

**Table 2: Stability test result of tetrodotoxin in acidic solution under different temperature conditions**

| Time | Content (%) | | | Remarks |
|---|---|---|---|---|
| | 6°C | 25°C | 40°C | |
| 0 day | 99.2 | 99.20 | 99.20 | Solubilizer is acetic acid solution with pH 4.05. |
| 5 days | - | 97.88 | 91.58 | |
| 10 days | - | 97.54 | 86.30 | |
| 30 days | 96.99 | 93.88 | 69.57 | |

For unstable drugs in water solution, a regular method used in this field is freeze-dried preparation to get stable injection preparation. For instance, in CN 1835754A a stable freeze-dried powder tetrodotoxin was released where the dosage was selected from disaccharide such as lactose, sucrose, maltose and cellobiose, as well as proteoglycan including polyglucose, dextran or its derivatives including hydroxyethyl starch and hydroxypropyl-cyclodextrin stabilizers and taking 5-100mg for each dose. Furthermore, the preparation also comprises solubilizer such as citric acid, tartaric acid, malic acid or lactobionic acid. The usage of each dose is 0.00005-0.0005mg. In CN 1835754A it is pointed out that using citrate or mannitol as excipient, the obtained freeze-dried tetrodotoxin is unstable and the concentration of tetrodotoxin would gradually degrade in the storage process.

We still need to research and produce safe, stable, and quality products for medicinal use which can be stored for a long period of time for tetrodotoxin clinical use.

### SUMMARY OF THE INVENTION

To obtain stable tetrodotoxin, the applicant has carried out intensive research in freeze-dried powder preparation and the producing technology thereof. Result shows that formation framework of excipient, tetrodotoxin stabilizer, solubilizer and the pH range of prescription solution, the technology of freezing, sublimation and drying under vacuum, and the control of tetrodotoxin water content can significantly influence the stability of powder preparation, particularly, the water absorbance of the powder formation framework carrier. Throughout the long storing process of the highly water absorbent supplemental materials the water content of the preparation would rise gradually; and with increase in water content, the content of tetrodotoxin in the preparation would gradually drop.

Thanks to voluminous experimental research, the inventor finds out that using lixiviated sodium chloride or mannitol as freeze-dried powder formation framework supplemental materials, and using dextran 20 or trehalose as the tetrodotoxin stabilizer while adjusting the pH value to 3.5-4.5 hours prior to freeze-drying, the tetrodotoxin freeze-dried powder preparation looks like a white and loose cake. Such dosage preparation is accurate, looks fair in outward appearance, has stable quality and is safe and conform to the requirements of injection use on the human body.

Hence, this invention has provided a tetrodotoxin freeze-dried powder preparation for safe injection of the human body wherein the preparation contains tetrodotoxin, solubilizer, freeze-dried excipient and stabilizer. The purity of the said tetrodotoxin is greater than 96%, preferably 98%∼99.8%. The freeze-dried excipient is sodium chloride or mannitol, or their composite. The stabilizer is dextran or trehalose, or their composite, and the solubilizer is citric acid.

In this invention, the tetrodotoxin freeze-dried powder preparation is preferably having a ratio of tetrodotoxin:excipient:stabilizer at 1: 150-3000: 50-500 or 50-6000.

In this invention, the tetrodotoxin freeze-dried powder preparation should have a content of tetrodotoxin at 0,1∼20.0µg/dose, preferably 0.5∼20.0µg/dose, and more preferably 0.5∼12.0µg/dose.

In this invention, the tetrodotoxin freeze-dried powder preparation should have a content of sodium chloride in excipient at 1.0∼30mg/dose, preferably 5.0∼30mg/dose, and more preferably 5.0∼20mg/dose.

In this invention, the tetrodotoxin freeze-dried powder preparation should have a content of mannitol in excipient at 1.0∼30mg/dose, preferably 1.0∼20mg/dose, and more preferably at 3.0∼10mg/dose.

In this invention, the tetrodotoxin freeze-dried powder preparation should have a content of dextran in stabilizer at 0.5∼5.0mg/dose, preferably 2.0∼5.0mg/dose, and more preferably 3.0∼5.0mg/dose.

In this invention, the tetrodotoxin freeze-dried powder preparation should have a content of trehalose in stabilizer at 0.5∼60mg/dose, preferably 2.0∼60mg/dose, and more preferably 10∼60mg/dose.

In this invention, the tetrodotoxin freeze-dried powder preparation should have a content of citric acid at 0.001∼0.080mg/dose, preferably 0.010∼0.080mg/dose, and more preferably 0.020∼0.060mg/dose.

In this invention, the tetrodotoxin freeze-dried powder preparation preferably should have function modulator, preferably lidocaine hydrochloride.

In this invention, the tetrodotoxin freeze-dried powder preparation preferably should fill in noble gas such as high purity nitrogen or high purity carbon dioxide.

Preferably the tetrodotoxin freeze-dried powder preparation of this invention should be by muscle or subcutaneous injection, and in using bacteria-free injection water for dissolution, the amount of water to be used should be 0.5∼2.0ml/dose.

Furthermore, this invention also has provided the method for producing tetrodotoxin freeze-dried powder preparation and the method comprises following steps:
(1) Directly dissolve a fixed volume of tetrodotoxin into the solubilizer solution, adjust the pH value to 3.0∼6.0, preferably pH 3.5-4.5 and filter to eliminate the pyrogen.
(2) Directly dissolve the freeze-dried excipient, stabilizer and any selected function modulator in the bacteria-free injection water, add activated carbon and stir for 30 minutes, then filter to eliminate the pyrogen.
(3) Evenly mix the solutions obtained from (1) and (2), filter to remove bacteria and pour into a cillin bottle at a volume, freeze dry under vacuum, fill in noble gas, compress and cover with a lid to obtain the freeze-dried powder preparation product.

Follow step 1 of this invention method, preferably filter through ultrafiltration.

Follow step 2 of this invention method, the quantity of activated carbon used preferably be 0.1∼6.0g/100ml.

Follow step 3 of this invention method, filter through the .05µm∼0.20µm submicron millipore membrane or charged millipore filter membrane.

### BRIEF DESCRIPTION OF DRAWINGS

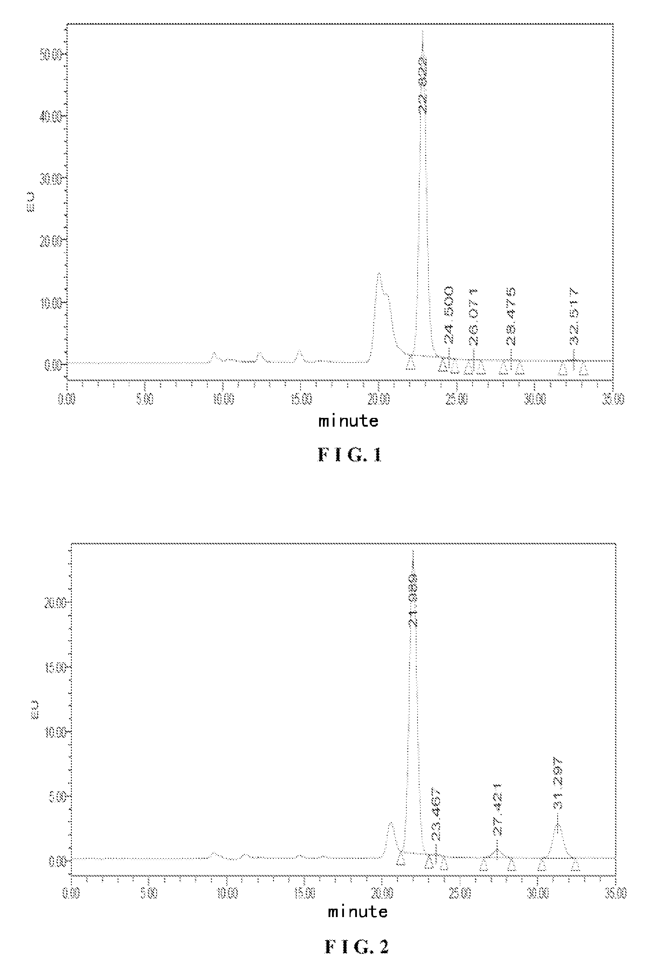

FIG. 1 HPLC fluorescent testing chromatography of tetrodotoxin injection solution at 40°C for 0 day.
FIG. 2 HPLC fluorescent testing chromatography of tetrodotoxin injection solution at 40°C for 10 days.

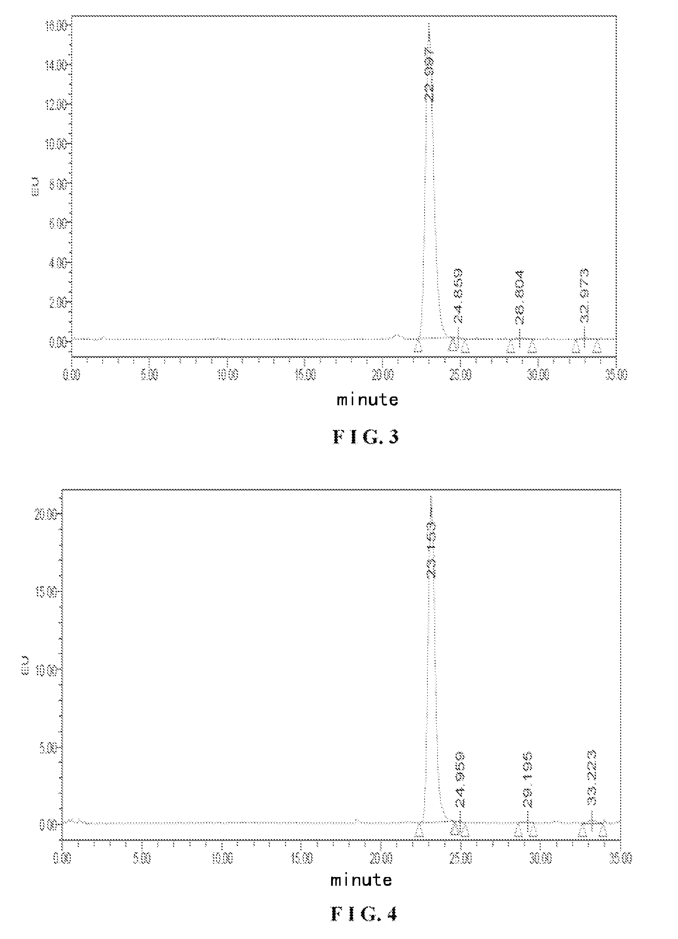

FIG. 3 HPLC fluorescent testing chromatography of prescription A1 tetrodotoxin freeze-dried powder preparation at 40°C for 0 day.
FIG. 4 HPLC fluorescent testing chromatography of prescription A1 tetrodotoxin powder preparation at 40°C for 10 days.

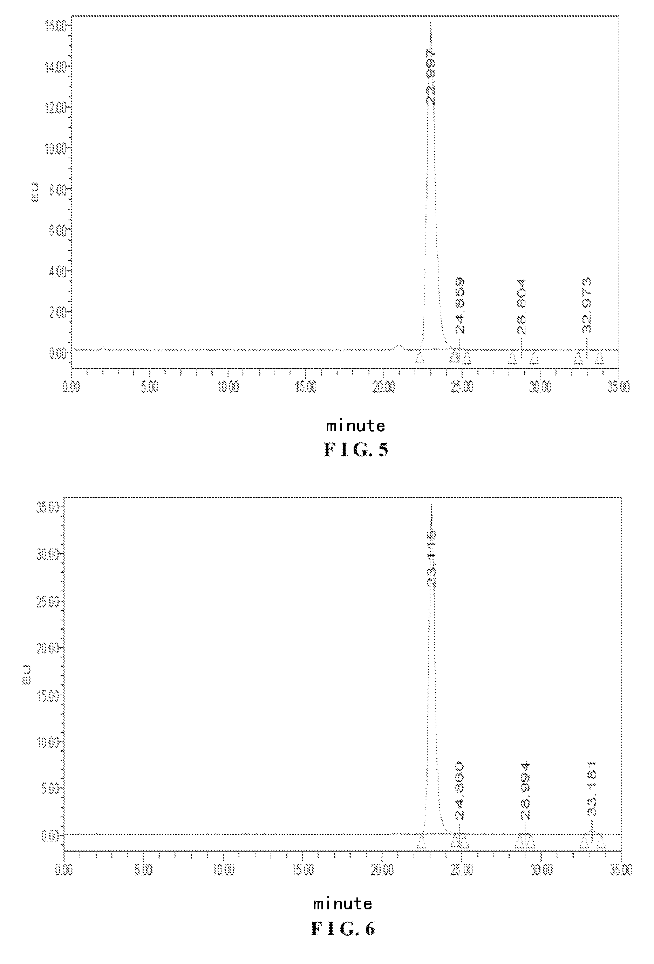

FIG. 5 HPLC fluorescent testing chromatography of the prescription B1 tetrodotoxin freeze-dried powder preparation at 40°C for 0 day.
FIG. 6 HPLC fluorescent testing chromatography of the prescription B1 tetrodotoxin freeze-dried powder preparation at 40°C for 10 days.

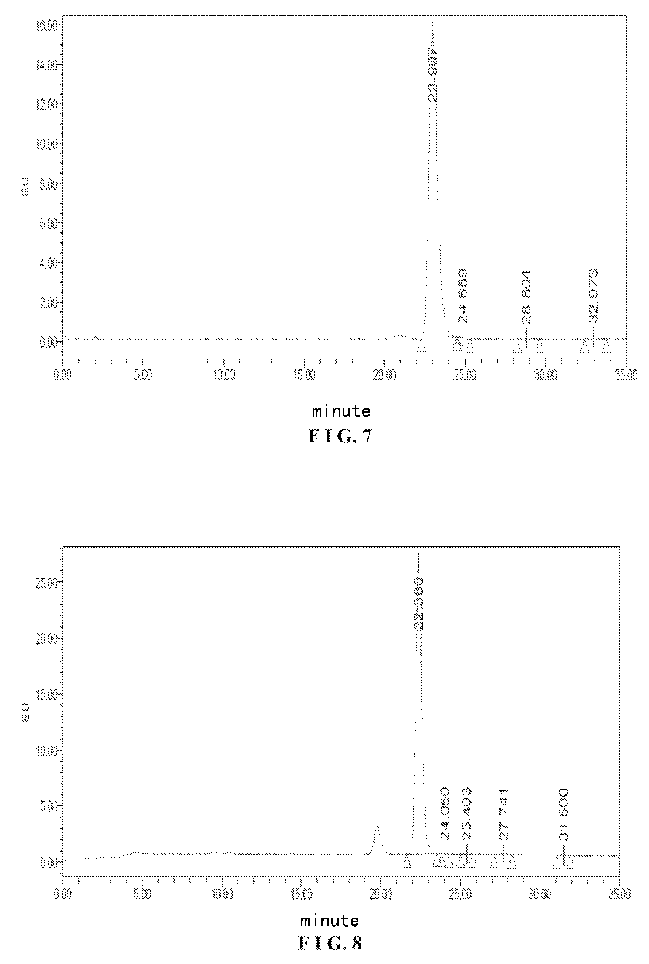

FIG. 7 HPLC fluorescent testing chromatography of the prescription C1 tetrodotoxin freeze-dried powder preparation at 40°C for 0 day.
FIG. 8 HPLC fluorescent testing chromatography of the prescription C1 tetrodotoxin freeze-dried powder preparation at 40°C for 10 days.

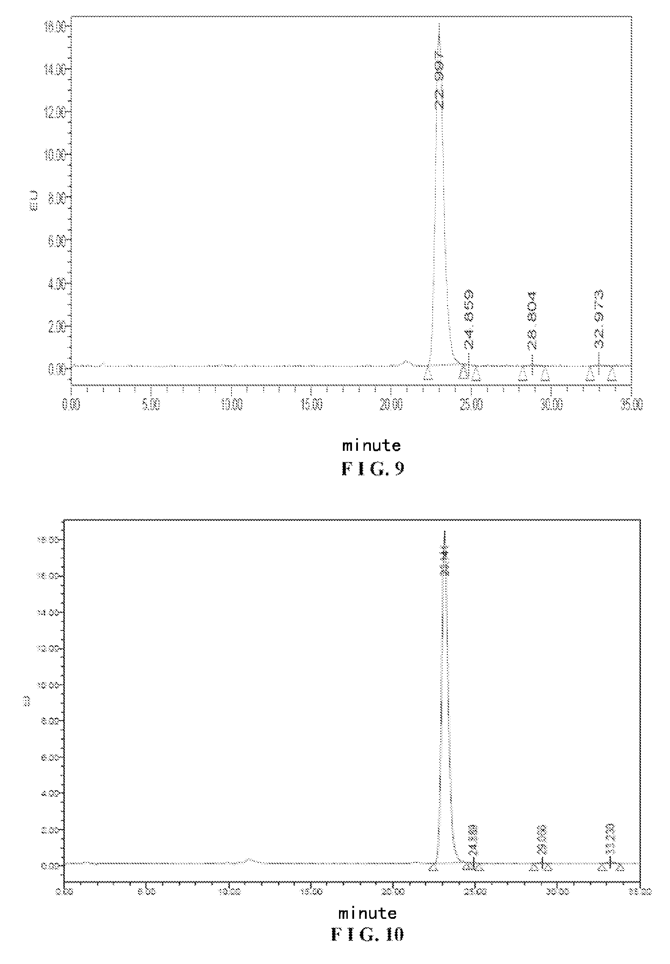

FIG. 9 HPLC fluorescent testing chromatography of the prescription D5 tetrodotoxin freeze-dried powder preparation at 40°C for 0 day.
FIG. 10 HPLC fluorescent testing chromatography of the prescription D5 tetrodotoxin freeze-dried powder preparation at 40°C for 10 days.

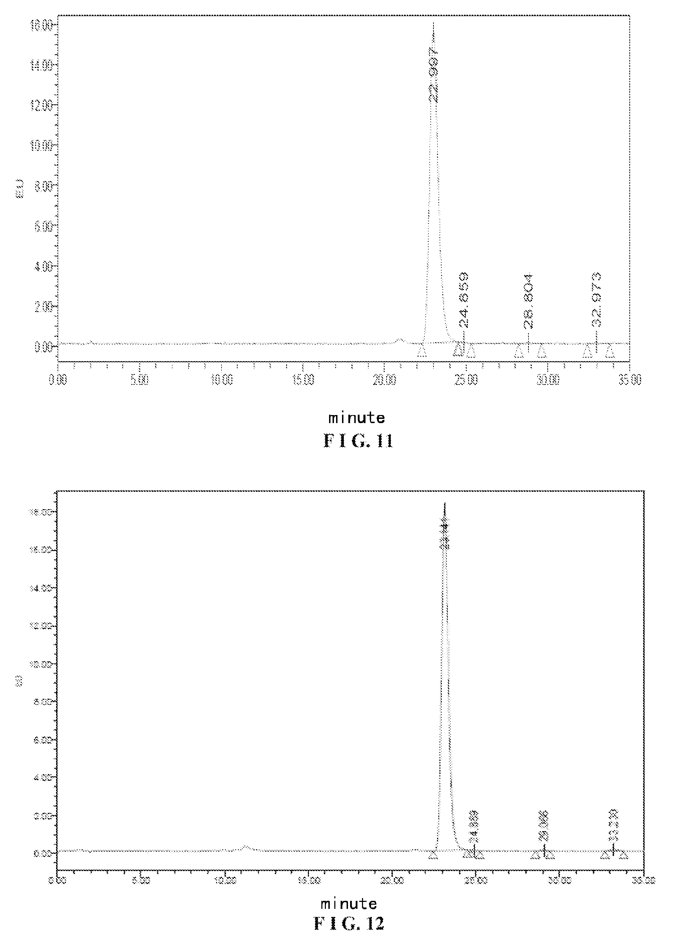

FIG. 11 HPLC fluorescent testing chromatography of the prescription E5 tetrodotoxin freeze-dried powder preparation at 40°C for 0 day.
FIG. 12 HPLC fluorescent testing chromatography of the prescription E5 tetrodotoxin freeze-dried powder preparation at 40°C for 10 days.

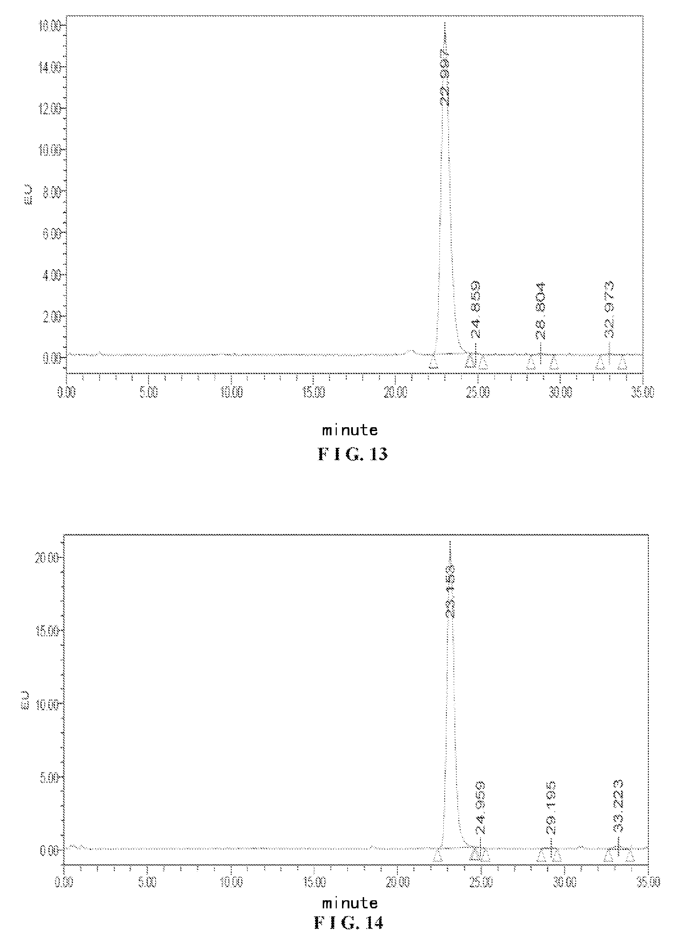

FIG. 13 HPLC fluorescent testing chromatography of the prescription F5 tetrodotoxin freeze-dried powder preparation at 40°C for 0 day.
FIG. 14 HPLC fluorescent testing chromatography of the prescription F5 tetrodotoxin freeze-dried powder preparation at 40°C for 10 days.

### DETAIL DESCRIPTION OF THE PREFERRED EMBODIMENTS

In this manual, we used the high performance liquid chromatography (Chinese Pharmacopoeia 2005 Editionm, Volume II, Appendix VII) to test the content of tetrodotoxin in the freeze-dried powder preparation of this invention. In the recorded chromatograph under the content test item, the retention time of the sample solution peak is consistent with the retention time of the reference solution peak.

The used chromatograph conditions are as follows: Use diatomaceous silica as filler and use phosphate of the Octanesulfonic acid as the buffer solution in the mobile phase. Its flow speed is 0.3ml/minute, the excitation wavelength is 365nm and emission wavelength is 510nm. Its column derivative is 4mol/L of sodium hydroxide solution with a flow speed of 0.2-0.5ml/minute, and the column derivative temperature is 100°C∼140°C. Counted on tetrodotoxin peak the theoretical plate number is not less than 2000.

The specific testing method is as follows: Take the tetrodotoxin freeze-dried powder preparation (which contains 10µg tetrodotoxin) of this invention, add precisely 2.0ml water to dissolve it, and precisely measure 20µl and introduce sample under the prepared chromatograph conditions and record the fluorescent testing chromatography. Also, prepare appropriate reference volume of tetrodotoxin. Use the same method for testing. Count the tetrodotoxin content of each dose/bottle using the peak area of external labeling. Tetrodotoxin conforms to requirements of freeze-dried preparation if its content is 90%∼110% of its labeled content.

Testing of related materials: Take tetrodotoxin freeze-dried preparation of this invention, add water to produce sample solution with 1ml containing 20µg of tetrodotoxin. Precisely measure 1.0ml and place in a 25ml volumetric flask, dilute to the mark, shake to mix evenly and use it as a reference solution. Precisely measure 20µl each of sample solution and reference solution, and pour respectively into the liquid chromatography, and record the fluorescent testing chromatography until its main content peak retention time doubles. The sample solution conforms to medicinal requirements if the sum of the impurities peak area is not greater than the reference solution peak area in the chromatography.

In this invention, we used the high performance liquid chromatography testing method to test the content of tetrodotoxin and related materials. Compared to the regular high performance liquid chromatography ultraviolet testing method, the HPLC fluorescent testing method is more sensitive and accurate, thereby they can better be used for testing the stability of tetrodotoxin products. For instance, for tetrodotoxin, the lowest testing limit for using HPLC ultraviolet testing method is 8.14ng and for using HPLC fluorescent method is 0.40ng; that means, fluorescent testing is 20 times lower than the ultraviolet method. Moreover, for tetrodotoxin preparation, the set amount for the HPLC ultraviolet method is 20.26ng while that for HPLC fluorescent method is 0.81ng, in other words, the set amount for the fluorescent method is 25 times lower than the ultraviolet method.

Generally, the regular specification of the injection tetrodotoxin product is 10µg/bottle. Upon dissolving the sample, the sample concentration for testing is 5µg /ml- 20µg/ml. If the sample amount is 20µl and the sample contains 1% impurities and the impurities amount is only 1-4ng; this falls below the testing limit using the liquid chromatography method. If the sample contains 2% impurities the impurities amount is only 2-8ng; that is also below the testing limit of the liquid chromatography ultraviolet testing method. Thus, we can see that when the product has less than 5% impurities, it is possible that because of the testing limit the liquid chromatography testing method may result in testing deviation. On the contrary, the testing limit for liquid chromatography fluorescent testing method is 0.40ng and this completely meets the testing requirements.

Using aforementioned testing conditions, the applicant can carry out research of various factors that can influence the stability of tetrodotoxin freeze-dried powder preparation.

### Screening of tetrodotoxin freeze-dried powder excipient and stabilizer

The clinical usage of tetrodotoxin is measured by milligram, therefore, it is necessary to add excipient as supplemental material for formation framework of the freeze-dried powder preparation. Concomitantly, the screening of stabilizer is also of vital importance because of requirement for the stability of tetrodotoxin powder preparation. Through voluminous experiments, the applicant has found out that, the best result can be obtained by using sodium chloride and/or mannitol as the supplemental excipient for tetrodotoxin freeze-dried powder preparation, and selecting dextran 20 or trehalose as stabilizer. Tables 3 and 4 below have summarized the stability testing results for the prescription of tetrodotoxin freeze-dried powder preparation under 40°C by using excipient which contains sodium chloride and/or mannitol and stabilizer which contains dextran 20 or trehalose.

**Table 3: Tetrodotoxin freeze-dried powder preparation prescription**

| Prescription | 1 | 2 | 3 | 4 | 5 | 6 |
|---|---|---|---|---|---|---|
| Tetrodotoxin (µg) | 10 | 10 | 10 | 10 | 10 | 10 |
| Supplemental excipient | Sodium chloride (9mg) | Sodium chloride (9mg) | Mannitol (6mg) | Mannitol (10mg) | Sodium chloride (9mg) Mannitol (6mg) | Sodium chloride (9mg) |
| Stabilizer | Dextran 20(4mg) | | Dextran 20 (4mg) | | Dextran 20 (4mg) | Trehalose (4mg) |

**Table 4: Stability testing result of different prescriptions of tetrodotoxin freeze-dried powder preparation under 40°C**

| Prescription | Outward appearance | 0 day | | 5 days | | 10 days | |
|---|---|---|---|---|---|---|---|
| | | Content (%) | Related material | Content (%) | Related material | Content (%) | Related material |
| 1 | Plump, white, cake shape | 100 | <Reference main peak area | 99.30 | <Reference main peak area | 98.70 | <Reference main peak area |
| 2 | White, cake shape, under 40°C with no significant change in 10 days | 100 | <Reference main peak area | 97.84 | >Reference main peak area | 97.03 | >Reference main peak area |
| 3 | White, cake shape, under 40°C with no significant change in 10 days | 100 | <Reference main peak area | 99.06 | <Reference main peak area | 98.41 | <Reference main peak area |
| 4 | White, cake shape, under 40°C with no significant change in 10 days | 100 | <Reference main peak area | 95.56 | >Reference main peak area | 92.26 | >Reference main peak area |
| 5 | White, cake shape, under 40°C with no significant change in 10 days | 100 | <Reference main peak area | 98.96 | <Reference main peak area | 98.59 | <Reference main peak area |
| 6 | White, cake shape, under 40°C with no significant change in 10 days | 100 | <Reference main peak area | 99.37 | <Reference main peak area | 98.71 | <Reference main peak area |

The outward appearance of the mannitol product used as excipient looks good with little moisture absorbency. Dextran has significant protective function for tetrodotoxin freeze-dried preparation but its water absorbency is strong. To control the water content of freeze-dried powder product with plump freeze-dried formation framework, the content of dextran 20 in the preparation of this invention is 4mg/dose and mannitol 6mg/dose. On the other hand, if lixiviated modulator sodium chloride is used as excipient formation framework supplemental material and dextran 20 or trehalose used as stabilizer to form the prescription, the water content of the product is low and its outward appearance looks plump. In practical applications, adding water would swiftly dissolve. Under 40°C high temperature, 10 day stability test meets medicinal requirements.

### Selection of solubilizer and prescription pH value

Tetrodotoxin is a nonprotein marine neurotoxin with a relative molecular mass of 319.27 and often exists in the form of amphiphatic molecule. The guanidyl is a function base in the structure required by its activeness. It does not dissolve in water or organic solvent. It is easily dehydrated or decomposed when strong acid and strong alkaline is encountered. It is relatively stable in weak acidic solution, therefore, the freeze-dried powder preparation produced from tetrodotoxin should select appropriate acidic solvent as the solubilizer. The applicant has referenced the Chinese Pharmacopoeia 2005 edition, the Medicinal Supplemental Mateials Handbook and the Complete Works of Chinese Medicinal Supplemental Materials. We have analyzed and selected the frequently used injection standard weak acidic materials such as citric acid, acetic acid, ascorbic acid, phosphoric acid and monosodium phosphate in injection. Based on the Guiding Principle for Chemical and Pharmaceutical Preparation Research Technology for priority selection, acetic acid is a volatile acid and has volatility loss during the freeze-drying process. This does not favor pH control in dissolution of the powder preparation by adding water. The chemical and physical properties of ascorbic acid are not stable enough under light and room temperature. Monosodium phosphate is acid salt which is weakly acidic. Its pH value is only 4.56 under 5% concentration. Its pH adjustment range is narrow and is not suitable for acid solvent in tetrodotoxin freeze-dried powder. Citric acid is nonvolatile and weakly acidic with a comparatively wider space for pH adjustment. Henceforth, through analysis and comparison we have selected citric acid as the solubilizer in tetrodotoxin freeze-dried powder of this invention. For freeze-dried powder preparation in this invention, it preferably uses 0.001mg to 0.080mg per dose as solubilizer citric acid.

The pH value of the injection solution is one of the critical factors closely related to the stability of effective medicinal content. The applicant used a set prescription for tetrodotoxin freeze-dried powder and used 0.1% citric acid to adjust different pH values. After freeze-drying the powder products, study its outward appearance, dissolution and pH value and stability. Main stability test: Place the product under 40°C and inspect samples at 0, 5 and 10 days, and use the liquid chromatography fluorescent testing method for testing. The content and related materials of tetrodotoxin are calculated by the area normalization method. Refer to Table 5 for the experimental result.

**Table 5: Influence of powder preparation pH value on tetrodotoxin stability**

| pH | 0 day | | 5 days | | 10 days | |
|---|---|---|---|---|---|---|
| value | Tetrodotoxin content (%) | Related materials | Tetrodotoxin content (%) | Related materials | Tetrodotoxin content (%) | Related materials |
| 6.0 | 99.30 | <Reference solution peak area | 97.21 | >Reference solution peak area & appear new impurities peak | 96.30 | >Reference solution peak area & appear new impurities peak |
| 5.0 | 99.30 | <Reference solution peak area | 97.58 | >Reference solution peak area & appear new impurities peak | 96.56 | >Reference solution peak area & appear new impurities peak |
| 4.5 | 99.30 | <Reference solution peak area | 98.71 | <Reference solution peak area | 98.37 | <Reference solution peak area |
| 4.0 | 99.30 | <Reference solution peak area | 98.62 | <Reference solution peak area | 98.41 | <Reference solution peak area |
| 3.5 | 99.30 | <Reference solution peak area | 98.24 | <Reference solution peak area | 98.10 | <Reference solution peak area |

From aforementioned experimental results we can see that pH of tetrodotoxin powder preparation solution has significant influence on the stability of tetrodotoxin. Under 40°C and the pH value greater than 5 and maintain for 10 days, the related materials degraded from tetrodotoxin is greater than the reference solution peak area and appear a new impurities peak.

After repeated experiments, the applicant found out that the preferable pH value of the tetrodotoxin freeze-dried powder preparation is 3.5-4.5, and the best pH value is around 4.0.

### Influence of different water content of tetrodotoxin freeze-dried powder product on stability

The applicant has carried out research on the influence of different water content of tetrodotoxin freeze-dried powder product on stability. Refer to Table 6 for results.

**Table 6: Test result of influence of different water content of tetrodotoxin freeze-dried powder product under 40°C on stability**

| Batch no. | | 1 | 2 | 3 |
|---|---|---|---|---|
| Product water content (%) | | 8.56 | 5.23 | 2.50 |
| Content limit (%) | | 90∼100 | 90∼100 | 90∼100 |
| 0 day | Content (%) | 100 | 100 | 100 |
| | Related materials | <Reference solution peak area | <Reference solution peak area | <Reference solution peak area |
| 5 days | Content (%) | 97.65 | 98.43 | 99.21 |
| | Related materials | >Reference solution main peak area | <Reference solution main peak area | <Reference solution main peak area |
| 10 days | Content (%) | 95.28 | 97.89 | 98.64 |
| | Related materials | >Reference solution main peak area | >Reference solution main peak area | <Reference solution main peak area |
| | Outward appearance | | | |

Experiment shows: The outward appearance of product would shrink under 40°C and stability is comparatively poor when the water content of the freeze-dried powder product is greater than 5%. The less water content of the freeze-dried product, the more stable would be tetrodotoxin of product. The water content of tetrodotoxin freeze-dried powder preparation should preferably be controlled under 3%.

### Choice of freeze-drying technology

The freeze-drying technology of tetrodotoxin powder preparation primarily comprise of pre-freezing, main drying and rear drying stages. We have designed five freeze-dried solutions for experiment (refer to Table 7) according to the freeze-drying technology while screening the common features, excipient and stabilizer of the prescription products. Comparative analysis of the testing results, we have ascertained the control parameters for the best freeze-drying technology of this product (refer to Table 8).

**Table 7: Choice of freeze-drying technology of tetrodotoxin freeze-dried powder preparation**

| Solution | Technology |
|---|---|
| Solution 1 | -35°C pre-freeze 2.5 hours, -10°C vacuum low temperature drying 15 hours, 40°C vacuum drying for 4 hours |
| Solution 2 | -35°C pre-freeze 2.5 hours, vacuum dry for 8 hours under -10°C temperature, 10°C vacuum dry for 3 hours, 20°C vacuum dry for 3 hours, 30°C vacuum dry for 4 hours |
| Solution 3 | -35°C pre-freeze 2.5 hours, vacuum dry for 8 hours under -10°C temperature, 10°C vacuum dry for 3 hours, 20°C vacuum dry for 3 hours, 35°C vacuum dry for 4 hours |
| Solution 4 | -35°C pre-freeze 2.5 hours, vacuum dry for 8 hours under -10°C temperature, 10°C vacuum dry for 3 hours, 20°C vacuum dry for 3 hours, 40°C vacuum dry for 4 hours |
| Solution 5 | -35°C pre-freeze 2.5 hours, vacuum dry for 8 hours under -10°C temperature, 10°C vacuum dry for 3 hours, 20°C vacuum dry for 3 hours, 30°C vacuum dry for 4 hours |

**Table 8: Result evaluation of freeze-drying technology of tetrodotoxin freeze-dried powder preparation**

| Solution | Result evaluation |
|---|---|
| Solution 1 | Formation framework outward appearance poor, tetrodotoxin content of product noticeably drops. |
| Solution 2 | Formation framework outward appearance conforms to requirements, water content high, tetrodotoxin content of product drops. |
| Solution 3 | Formation framework outward appearance comparatively fair, water content slightly higher, tetrodotoxin content of product slightly drops. |
| Solution 4 | Formation framework outward appearance comparatively fair, water content <3.0%, tetrodotoxin of product in technology process did not drop. |
| Solution 5 | Formation framework outward appearance comparatively fair, water content <2.0%, tetrodotoxin content of product did not drop throughout the technology process. |

Experiment of solution 1 found out that when the product is being pre-freezed to -10°C and vacuum dry for 15 hours, and the drying temperature directly rises to 40°C, the tetrodotoxin of the product would significantly drop. This shows that the design of the freeze-drying technology has significant influence on the quality of the tetrodotoxin freeze-dried powder preparation. Through passing the optimal drying curve of solution 4, the main drying stage uses rising temperature step by step to a rear drying stage of 40°C and obtain low water content of product. The freeze-drying technology process has no influence on the content of tetrodotoxin in the product. Therefore, we have selected solution 4 as the preferable freeze-drying technology for this product.

### Other practical embodiments

### Embodiment 1: Comprises sodium chloride as the excipient, dextran 20 as the stabilizer and citric acid as the solubilizer for tetrodotoxin freeze-dried powder preparation.

Preparation has following compositions for tetrodotoxin freeze-dried powder preparation:

| Composition | Tetrodotoxin (purity>98%) (µg/dose) | Sodium chloride (mg/dose) | Dextran 20 (mg/dose) | Prior to freeze-drying, use citric acid to adjust the solution pH value to |
|---|---|---|---|---|
| Prescription A1 | 10 | 9 | 4.0 | 4.0 |
| Prescription A2 | 5 | 9 | 4.0 | 3.5 |
| Prescription A3 | 10 | 9 | 4.0 | 3.8 |
| Prescription A4 | 15 | 9 | 4.5 | 4.0 |
| Prescription A5 | 8 | 9 | 4.5 | 4.2 |
| Prescription A6 | 20 | 9 | 5.0 | 3.5 |

Preparation method: Take a prescription amount of tetrodotoxin, and use 10ml 0.1% citric acid solution to dissolve it, add a prescription amount of sodium chloride and add injection water to dilute to approximately 250ml. Use 0.1% citric acid solution to adjust to the specified pH value, filter with ultrafiltration to eliminate the pyrogen and obtain Group A solution. Also, take a prescription amount of dextran 20, add 200ml injection water to dissolve it, use 0.1% citric acid solution to adjust to the specified pH value, then add 0.1%∼1.0% of a ratio of weight:volume of activated carbon and keep it at a temperature of 60°C while stirring for 30 minutes; afterwards, filter to remove carbon and the pyrogen and cool to room temperature to obtain a group B solution. Evenly mix the aforementioned groups A and B solutions, use injection water to obtain a volume of 500ml, then use 0.22µm millipore membrane to filter, take samples to check the pH value, clarity, and content to ensure conformance and carry out bacteria-free packaging. After pre-freezing at -35°C for 2∼6 hours, mainly dry at -10°C∼20°C for 10∼20 hours, and later dry at 20°C∼50°C for 6∼10 hours to get the product. The outward appearance of the product is a white cake shape article.

From aforementioned tetrodotoxin freeze-dried powder preparation, you may also select any lidocaine hydrochloride with function modulator with a quantity of 5mg/dose.

### Embodiment 2: Tetrodotoxin freeze-dried powder preparation having mannitol as the excipient, dextran 20 as the stabilizer with citric acid as the solubilizer.

Preparation has following compositions for tetrodotoxin freeze-dried powder preparation:

| Composition | Tetrodotoxin (purity>99%) (µg/dose) | Sodium chloride (mg/dose) | Dextran 20 (mg/dose) | Prior to freeze-drying, use citric acid to adjust the solution pH value to |
|---|---|---|---|---|
| Prescription B1 | 10 | 6 | 4.5 | 3.8 |
| Prescription B2 | 20 | 15 | 5 | 3.5 |
| Prescription B3 | 5 | 6 | 5 | 3.0 |
| Prescription B4 | 3 | 6 | 4 | 4.0 |
| Prescription B5 | 15 | 6 | 5 | 4.2 |
| Prescription B6 | 12 | 10 | 5 | 4.5 |

Preparation method: Take a prescription amount of tetrodotoxin, use 10ml 0.1% citric acid solution to dissolve it, add injection water to dilute to approximately 250ml. Use 0.1% citric acid solution to dilute to the specified pH value, and use the filter membrane to filter out the pyrogen and obtain the group A solution. Also, take a prescription amount of dextran 20 and mannitol, add 200ml injection water to dissolve it; use 0.1 % citric acid solution to adjust to the specified pH value, add 0.2% of a ratio of weight: volume of activated carbon and stir for 30 minutes at 60°C, filter out the carbon and eliminate pyrogen, and cool to room temperature to obtain a group B solution. Evenly mix the groups A and B solutions, use injection water to prepare a volume of 500 ml; use 0.22µm millipore membrane to filter, and take samples to test the pH value, clarity, and content to ensure conformance, and carry out bacteria-free packaging. Pre-freeze at -35°C for 2∼6 hours, carry out main drying at -10°C∼20°C for 10∼20 hours, and later dry at 20°C∼0°C for 6∼10 hours to obtain the product. The outward appearance of the product looks like a white cake shaped article.

From aforementioned tetrodotoxin freeze-dried powder preparation, you may also select any lidocaine hydrochloride with function modulator at a quantity of 3.0mg/dose. Embodiment 3: Tetrodotoxin freeze-dried powder preparation having sodium chloride as the excipient, trehalose as the stabilizer with citric acid as the solubilizer for textrodotoxin freeze-dried powder preparation.

Preparation has following compositions for tetrodotoxin freeze-dried powder preparation:

| Composition | Tetrodotoxin (purity>99%) (µg/dose) | Sodium chloride (mg/dose) | Dextran 20 (mg/dose) | Prior to freeze-drying, use citric acid to adjust the solution pH value to |
|---|---|---|---|---|
| Prescription C1 | 10 | 9 | 10 | 4.0 |
| Prescription C2 | 15 | 9 | 10 | 4.2 |
| Prescription C3 | 20 | 9 | 20 | 4.5 |
| Prescription C4 | 8 | 9 | 30 | 3.8 |
| Prescription C5 | 5 | 9 | 15 | 3.5 |
| Prescription C6 | 3 | 9 | 10 | 4.0 |

Preparation method: Take a prescription amount of tetrodotoxin, use 20ml 0.1% citric acid solution to dissolve it, add a prescription amount of sodium chloride and trehalose and add injection water to dilute to approximately 450ml. Use 0.1% citric acid solution to adjust to the specified pH value, and use the filter membrane to filter out the pyrogen, and use injection water to prepare a volume of 500ml, then use 0.22µm millipore membrane for filtration, take samples to check the pH value, clarity and content to ensure conformance, and carry out bacteria-free packaging. Pre-freeze at -35°C for 2∼6 hours, carry out main drying at -10°C∼20°C for 10∼20 hours, and later dry at 20°C∼0°C for 6∼10 hours to obtain the product. The outward appearance of the product looks like a white cake shaped article.

From aforementioned tetrodotoxin freeze-dried powder preparation, you may also select any lidocaine hydrochloride with function modulator at a quantity of 3.0mg/dose.

### Embodiment 4: Tetrodotoxin freeze-dried powder preparation having mannitol as the excipient, trehalose as the stabilizer with citric acid as the solubilizer for textrodotoxin freeze-dried powder preparation.

Preparation has following compositions for tetrodotoxin freeze-dried powder preparation:

| Composition | Tetrodotoxin (purity>99%) (µg/dose) | Mannitol (mg/dose) | Trehalose (mg/dose) | Prior to freeze-drying, use citric acid to adjust the solution pH value to |
|---|---|---|---|---|
| Prescription D1 | 3 | 5 | 5 | 3.8 |
| Prescription D2 | 5 | 5 | 10 | 4.0 |
| Prescription D3 | 8 | 5 | 20 | 4.0 |
| Prescription D4 | 15 | 5 | 40 | 3.5 |
| Prescription D5 | 10 | 5 | 30 | 4.0 |
| Prescription D6 | 20 | 5 | 60 | 4.5 |

Preparation method: Take a prescription amount of tetrodotoxin, add 20ml 0.1% citric acid solution, add a prescription amount of trehalose and add injection water to dilute to approximately 300ml. Use 0.1% citric acid solution to adjust to the specified pH value, and use ultrafiltration to filter out the pyrogen to obtain the group A solution. Also, take a prescription amount of mannitol, add 150ml injection water to dissolve it, use 0.1% citric acid solution to adjust to the specified pH value, add 0.1%∼1.0% of weight/volume ratio of activated carbon, stir for 30 minutes at 60°C, filter out the carbon, eliminate the pyrogen, and cool to room temperature to obtain the group B solution. Evenly mix the groups A and B solutions, use injection water to prepare a volume of 500ml, use 0.22µm millipore for filtration, take samples to check the pH value, clarity, and content to ensure conformance, and carry out bacteria-free packaging. Pre-freeze at -35°C for 2∼6 hours, then carry out main drying at -10°C∼20°C for 10∼20 hours, and then dry at 20°C∼50°C for 6∼10 hours to obtain the product. The outward appearance of the product looks like a white cake shaped article.

From aforementioned tetrodotoxin freeze-dried powder preparation, you may also select any lidocaine hydrochloride with function modulator at a quantity of 3.0mg/dose.

### Embodiment 5: Tetrodotoxin freeze-dried powder preparation containing mannitol and sodium chloride as excipients, trehalose as stabilizer which also contains citric acid as solubilizer.

Preparation has following compositions for tetrodotoxin freeze-dried powder preparation:

| Composition | Tetrodotoxin (purity>99%) (µg/dose) | Mannitol (mg/dose) | Sodium chloride (mg/dose) | Trehalose (mg/dose) | Prior to freeze-drying, use citric acid to adjust the solution pH value to |
|---|---|---|---|---|---|
| Prescription E1 | 3 | 5 | 9 | 5 | 3.5 |
| Prescription E2 | 5 | 5 | 9 | 10 | 4.0 |
| Prescription E3 | 8 | 5 | 9 | 15 | 3.8 |
| Prescription E4 | 15 | 5 | 9 | 30 | 4.0 |
| Prescription E5 | 10 | 5 | 9 | 20 | 4.0 |
| Prescription E6 | 20 | 5 | 9 | 40 | 4.5 |

Preparation method: Take a prescription amount of tetrodotoxin, add 20ml 0.1% citric acid solution to dissolve it, add a prescription amount sodium chloride and trehalose, add injection water to dilute it to approximately 300ml. Use 0.1% citric acid solution to adjust to the specified pH value, use ultrafiltration to filter out the pyrogen to obtain the group A solution. Also, take a prescription amount of mannitol, add 150ml injection water to dissolve it, then use 0.1% citric acid solution to adjust to the specified pH value, at a weight/volume ratio of 0.1%∼1.0% activated carbon stir for 30 minutes at 60°C, filter to remove carbon and eliminate pyrogen, cool to room temperature and obtain the group B solution. Evenly mix the groups A and B solutions, use injection water to prepare a volume of 500 ml, use 0.22µm millipore for filtration, take samples to check the pH, clarity, and content to ensure conformance, and carry out bacteria-free packaging. Pre-freeze at -35°C for 2∼6 hours, carry out main drying at -10°C∼20°C for 10∼20 hours, and then dry at 20°C∼50°C for 6∼10 hours, to obtain the product. The outward appearance of the product looks like a white cake shaped article.

From aforementioned tetrodotoxin freeze-dried powder preparation, you may also select any lidocaine hydrochloride with function modulator at a quantity of 3.0mg/dose.

### Embodiment 6: Tetrodotoxin freeze-dried powder preparation containing mannitol and sodium chloride as excipients, dextran 20 as stabilizer which also contains citric acid as solubilizer.

Preparation has following compositions for tetrodotoxin freeze-dried powder preparation:

| Composition | Tetrodotoxin (purity>99%) (µg/dose) | Mannitol (mg/dose) | Sodium chloride (mg/dose) | Dextran 20 (mg/dose) | Prior to freeze-drying, use citric acid to adjust the solution pH value to |
|---|---|---|---|---|---|
| Prescription F 1 | 3 | 3 | 9 | 4 | 3.5 |
| Prescription F2 | 5 | 3 | 9 | 4 | 3.5 |
| Prescription F3 | 10 | 3 | 9 | 4 | 4.0 |
| Prescription F4 | 15 | 3 | 9 | 5 | 4.2 |
| Prescription F5 | 10 | 3 | 9 | 4.5 | 4.0 |
| Prescription F6 | 20 | 3 | 9 | 5 | 4.5 |

Preparation method: Take a prescription amount of tetrodotoxin, add 20ml 0.1% citric acid solution to dissolve it, add a prescription amount of mannitol, sodium chloride, add injection water to dilute it to approximately 300ml. Use 0.1% of citric acid solution to adjust to the specified pH value, use ultrafiltration to filter out pyrogen to obtain the group A solution. Also, take a prescription amount of dextran 20, add 150ml injection water to dissolve it, use 0.1 % citric acid solution to adjust to the specified pH value, prepare a weight: volume ratio of 0.1 %∼1.0% activated carbon and stir for 30 minutes at 60°C, filter to remove carbon and eliminate pyrogen, and cool to room temperature to obtain the group B solution. Evenly mix the groups A and B solutions, use injection water to prepare a volume of 500 ml, use 0.22µm millipore for filtration, take samples to check the pH value, clarity, content to ensure conformance, and carry out bacteria-free packaging. Pre-freeze at -35°C for 2∼6 hours, and carry out main drying at -10°C∼20°C for 10∼20 hours, and then dry at 20°C∼50°C for 6∼10 hours to obtain the product. The outward appearance of the product looks like a white cake shaped article.

From aforementioned tetrodotoxin freeze-dried powder preparation, you may also select any lidocaine hydrochloride with function modulator at a quantity of 3.0mg/dose.

### Embodiment 7: Stability test

Take TTX freeze-dried powder preparation from above embodiment and carry out stability test at 40°C for 10 days. The specific operation of the test is as follows:

Place the various freeze-dried products in an experimental chamber at 40 ± 2°C and relative humidity at 75% ± 5% to test the stability of medicine. Take out the products after 10 days, and use the HPLC fluorescent method for testing. Changes in the purity of TTX show stability of the preparation.

In aforementioned stability test, the TTX of freeze-dried powder preparation of this invention has shown fairly good stability. The result of the preferable prescription tests is as follows:

**Table 9: Stability testing result of the preferable prescription of this invention of TTX freeze-dried powder preparation at 40°C for 10 days**

| Prescription | Outward appearance | 0 day | | 10 days | |
|---|---|---|---|---|---|
| | | Purity (%) | Related materials | Purity (%) | Related materials |
| A1 | White cake shaped article | 99.20 | <Reference solution main peak area | 98.56 | <Reference solution main peak area |
| B1 | White cake shaped article | 99.20 | <Reference solution main peak area | 98.71 | <Reference solution main peak area |
| C1 | White cake shaped article | 99.20 | <Reference solution main peak area | 99.00 | <Reference solution main peak area |
| D5 | White cake shaped article | 99.20 | <Reference solution main peak area | 99.06 | <Reference solution main peak area |
| E5 | White cake shaped article | 99.20 | <Reference solution main peak area | 99.10 | <Reference solution main peak area |
| F5 | White cake shaped article | 99.20 | <Reference solution main peak area | 98.63 | <Reference solution main peak area |

### Applied embodiments

There are a total of 80 examples of drug addicts who have applied preparation of this invention for abstinence treatment. There are 49 examples of male and 31 examples female with the oldest at 41 years of age and the youngest at 18 years of age. The longest has a drug history of 13 years and the shortest 2 years with the heaviest a daily drug taking of 2∼3g, and the lightest 0.1g. The types of drug abuse are heroin and heroin with sedative hynoptics and the ways of application include burning smell, muscle injection or subcutaneous injection of heroin. All 80 examples of patients conform to the DSM-III-R mental disorders caused by mental activated materials-a diagnosis standard of dependence on opiates, and testing of morphine in urine shows positive with no other serious bodily illness. Prior to treatment, only serious drug addicts have been selected for drug dependence abstinence. Drug usage of this preparation is 10ug-20ug per day for 3∼7 days.

The abstinence symptoms of opiate dependent patients differ upon stoppage of medicine. Patients are respectively given injection of this preparation in accordance with the seriousness of drug addiction and the extent of abstinence. Statistical results show that on first medication 95% of the patients have eliminated the abstinence symptoms within .5-3 hours, and the patients remain sober with no pain. The abstinence symptoms for the first time may disappear 6∼12 hours, and when symptoms reappeared, they show marked mitigation. For patients with a long history of addiction, we may give them 2 times injection of this preparation in 24 hours for the subcutaneous injection patients. All patients have lightly passed the most violent abstinence period under sober conditions with no pain. Patients are in good order with good appetite and rapidly restored bodily vitality upon elimination of the abstinence symptoms. Generally, it is unnecessary for further treatment after 3∼6 times of medication. No side effects or discomfort throughout the course of medication for all examples.

### Typical embodiments

Patient E.S., male, and has a drug addiction history of 10 years with average daily taking of 1.0g and the drug type is heroin and application is mainly by muscle or subcutaneous injection. The patient has stopped use of drug and voluntarily accepted treatment. At 19: 00 on the same day, abstinence symptoms appeared and at 21:00 the symptoms became more serious with running nose, tears, yawns, bone and muscle pain, shivering, abdominal pain, loose bowel, nausea, vomiting, goose pimple, erection of skin hair and curling of the body, etc. Immediately give muscle injection of this preparation when the abstinence symptoms reached "+ +"∼" + + +". After 0.5 hours, the abstinence symptoms have noticeably put under control, and the patient quietly slept after 2.5 hours with no pain. About 10 hours later, abstinence symptoms reappeared but with marked mitigation. At 9:30 the following day, the patient was again given injection of this preparation and the patient felt good after injection with no noticeable discomfort. At an interval of about 12 hours, the patient again had abstinence symptoms with loose bowel. Injection of this preparation was given at 21:00 and the patient felt fairly good and remained quiet throughout the night. Hereafter, he was given injection of this preparation every day until the patient felt marked improvement with no loose bowel whatsoever and appetite was restored and began normal defecation. Until the 5^{th} day, no abstinence symptoms appeared and the patient felt no discomfort. The treatment process lasted 5 days with a total of 6 times medication of this preparation.

## Claims

1. A method of preparing freeze-dried tetrodotoxin which contains tetrodotoxin, solubilizer, excipient and stabilizer, and the said tetrodotoxin should have a purity >96%, preferably 98%∼99.8%. The said excipient is sodium chloride or mannitol, or their composite; the stabilizer is dextran, trehalose or their composite; and the solubilizer is citric acid, and the freeze-dried method is by freezing, sublimation and drying under vacuum.

2. The preparation of tetrodotoxin of claim 1 for preparing freeze-dried tetrodotoxin, wherein the ratio of tetrodotoxin: excipient: stabilizer is 1: 150-3000: 50-500 or 50-6000.

3. The preparation of tetrodotoxin of claim 1, wherein the content of tetrodotoxin is 0,1∼20.0µg/dosage, preferably 0.5∼20.0µg/dosage, and more preferably 0.5∼12.0µg/dosage.

4. The preparation of tetrodotoxin of claim 1, wherein the content of sodium chloride in excipient is 1.0∼30mg/dose, preferably 5.0-30mg/dose, and more preferably 5.0-20mg/dose.

5. The preparation of tetrodotoxin of claim 1, wherein the content of mannitol in excipient is 1.0∼30mg/dose, preferably 1.0∼20mg/dose, and more preferably 3.0-10mg/dose.

6. The preparation of tetrodotoxin of claim 1, wherein the content of dextran in stabilizer is 0.5-5.0mg/dose, preferably 2.0∼5.0mg/dose, and more preferably 3.0∼5.0mg/dose.

7. The preparation of tetrodotoxin of claim 1, wherein the content of trehalose in stabilizer is 0.5∼60mg/dose, preferably 2∼60mg/dose, and more preferably 10∼60mg/dose.

8. The preparation of tetrodotoxin of claim 1, wherein the content of citric acid is 0.001∼0.080mg/dose, preferably 0.010∼0.080mg/dose, and more preferably 0.020∼0.060mg/dose.

9. The preparation of tetrodotoxin of any claims 1∼8, wherein the content also comprises lidocaine hydrochloride as function modulator.

10. The preparation of tetrodotoxin of any claims 1∼9, wherein the content also comprises noble gas such as high purity nitrogen or high purity carbon dioxide.

11. The preparation of tetrodotoxin of any claims 1∼10, wherein dispensation is by muscle or subcutaneous injection.

12. The method for preparation of tetrodotoxin as in any claims 1∼11 comprises of following steps:
(1) Directly dissolve a fixed amount of tetrodotoxin into any selected function modulator solution with solubilizer and adjust the pH value to 3.0∼6.0, preferably 3.5-4.5, and filter to eliminate the pyrogen.
(2) Directly dissolve the freeze-dried excipient and stabilizer into the bacteria-free injection water, add activated carbon and stir about 30 minutes, then filter to eliminate the pyrogen.
(3) Evenly mix the obtained solutions from (1) and (2), filter to eliminate bacteria, pour into a cillin bottle at a volume, vacuum freeze-dried, fill in noble gas, compress cover with lid, and you'll get freeze-dried powder prepared product.

13. The method as described in claim 12, wherein step 1 carries out filtration through a millipore filter.

14. The method as described in claims 12 or 13, wherein in step (2) the used quantity of activated carbon is 0.1∼6.0g/100ml.

15. The method as described in any claims 12-14, wherein in step 3 undergoes filtration of 0.05µm∼0.20µm with millipore membrane or charged millipore filter.

16. The vacuum freeze-dried method of claim 12, wherein in step 3 the pre-freeze temperature is between -20°C to -40°C for 2-3 hours; the main drying temperature at -10°C for 6-10 hours, at 10°C for 2-5 hours, and at 20°C for 2-5 hours; afterwards the drying temperature at 30-50°C for 4-10 hours.
